# FASCICULE DE BREVET EUROPEEN

(11) **EP 3 452 589 B1**
(45) Date de publication et mention de la délivrance du brevet: **06.01.2021**
(21) Numéro de dépôt: 16801505.5
(22) Date de dépôt: 25.11.2016
(51) Int. Cl.: C12N 15/10, C12M 1/34, C12Q 1/68

(54) **PROCEDE ET SYSTEME EXTRACTION MAGNETIQUE DE COMPOSANTS DANS UN ECHANTILLON LIQUIDE**
MAGNETISCHES EXTRAKTIONSVERFAHREN UND -SYSTEM VON BESTANDTEILEN IN EINER FLÜSSIGEN PROBE
METHOD AND SYSTEM FOR MAGNETIC EXTRACTION OF COMPONENTS IN A LIQUID SAMPLE

(30) Priorité: 03.05.2016 EP 16168001; 22.07.2016 EP 16180724
(43) Date de publication de la demande: 13.03.2019
(73) Titulaire: bioMérieux, 69280 Marcy-l'Étoile (FR)
(72) Inventeur: ALIX, David, 35140 Gosné (FR); MINASSIAN, Edgar, 69210 Lentilly (FR); RAYMOND, Jean-Claude, 69690 Bessenay (FR); WANDELS, Philippe, 69003 Lyon (FR)
(86) Numéro de dépôt international: PCT/EP2016/078905
(87) Numéro de publication internationale: WO 2017/190816

(56) Documents cités:
- EP-A1- 1 455 191
- EP-A1- 1 589 105
- EP-A1- 1 992 689
- EP-A2- 1 081 234
- WO-A1-03/006168
- WO-A1-2007/051859
- WO-A1-2014/072438
- WO-A1-2014/083165
- US-A- 5 234 809
- US-A1- 2011 076 205

## Description

### DOMAINE DE L'INVENTION

L'invention a trait au domaine de l'extraction de composants contenus dans une solution en utilisant des particules magnétiques.

L'invention trouve particulièrement application dans le domaine de la préparation d'échantillon biologique, notamment dans la mise en œuvre de diagnostic in vitro, par la capture d'analytes d'origine biologique (acides nucléiques, microorganismes, protéines, peptides, etc.) présents dans une solution.

### ETAT DE LA TECHNIQUE

A l'origine développée pour l'extraction d'acides nucléiques présents dans un échantillon biologique et décrite dans le document US 5 234 809, la technologie « BOOM® » consiste à introduire dans un échantillon liquide des particules magnétiques capables de se lier avec des composants d'intérêt, puis à séparer les particules magnétiques de l'échantillon à l'aide d'un ou plusieurs aimants. Les particules ainsi capturées peuvent alors subir un traitement ultérieur par exemple pour libérer leurs composants dans une solution de récupération. En raison de l'efficacité de cette technique, de nombreux dispositifs ont été développés et commercialisés, notamment pour l'ADN et TARN, ...), tant des dispositifs manuels (par exemple le NucliSENS-miniMAG® du demandeur) que des dispositifs automatisés (NucliSENS-easyMAG® du demandeur). Ces dispositifs automatisés souffrent cependant de diverses limitations.

Une première limitation concerne leur polyvalence et leur encombrement. En effet, ces dispositifs sont le plus souvent des automates lourds et encombrants qui sont conçus pour mettre en œuvre une séquence de traitements non modifiable par l'utilisateur. Un automate est ainsi conçu pour un seul type d'extraction, par exemple conçu pour la purification d'acides nucléiques mais incapable de mettre en œuvre une immuno-concentration magnétique.

Une deuxième limitation concerne les circuits d'injection et d'aspiration des différents liquides utilisés lors de l'extraction. Le nombre de liquides étant important, ceci implique des circuits également nombreux et/ou complexes. De plus, en raison de possibles contaminations, ces circuits d'injection/aspiration doivent être régulièrement nettoyés, ce qui implique la mise hors service des dispositifs. Une troisième limitation concerne les opérations de brassage qui sont mises en œuvre pour obtenir l'homogénéité de l'échantillon comprenant les particules magnétiques avant leur capture, pour maximiser la capture par ces dernières des analytes d'intérêt ou pour laver efficacement les particules magnétiques. Ce type de brassage nécessite usuellement des mécanismes complexes, par exemple à base d'aimants mobiles qui mettent en mouvement les particules magnétiques.

La quatrième limitation concerne les différents liquides utilisés lors de l'extraction. Usuellement, les étapes mises en œuvre pour l'extraction sont réalisées dans un ou à partir d'un seul récipient. De fait, ce récipient fixe un volume identique pour tous les liquides en jeu (e.g. l'échantillon, les différentes solutions de lavage, la solution d'élution, etc .), ce qui limite l'efficacité globale du processus d'extraction. En effet, certains traitements (e.g. le lavage) nécessitent de grands volumes pour être pleinement efficaces alors que d'autres traitements se contentent d'un petit volume de liquide (e.g. l'élution).

### EXPOSÉ DE L'INVENTION

Le but de la présente invention est de proposer un procédé d'extraction de composants dans un échantillon liquide à l'aide de particules magnétiques qui offre une grande liberté dans le choix des volumes liquides, en particulier jusqu'à 10 ml, utilisés lors de l'extraction.

A cet effet, l'invention a pour objet un procédé d'extraction de composants contenus dans un échantillon biologique sous forme liquide, lesdits composants étant aptes à se fixer sur des particules magnétiques, le procédé comprenant :
- une phase de mélange de l'échantillon avec les particules magnétiques;
- une phase d'aspiration du mélange depuis un puits dans un cône de pipette tubulaire comprenant une pointe destinée au pipetage de liquide;
- une phase de capture des particules magnétiques sur une paroi interne du cône de pipette :
- en appliquant un premier champ magnétique au cône de pipette, ledit champ étant apte à attirer et maintenir les particules magnétiques dans une zone prédéterminée du cône de pipette, dite de « capture », au-dessus de la pointe de celui-ci ;
- et en appliquant au moins un cycle d'aspiration et de refoulement du mélange contenu dans le cône de pipette dans un puits;
- au moins une phase de lavage des particules capturées sur la paroi interne du cône de pipette en :
- refoulant le mélange contenu dans le cône de pipette ; et
- en appliquant depuis un puits contenant une solution de lavage au moins un cycle d'aspiration et de refoulement de la solution de lavage dans le cône de pipette ;
caractérisé en ce que le procédé comprend en outre :
- une phase de migration des particules magnétiques sur la paroi interne du cône de pipette, depuis la zone de capture jusqu'à la pointe du cône de pipette, en réalisant un déplacement relatif du cône de pipette par rapport au premier champ magnétique;
- et une phase de transfert desdites particules magnétiques ayant migré dans la pointe du cône de pipette dans un puits de récupération contenant une solution,
- au moins une phase de lavage des particules capturées sur la paroi interne du cône de pipette, préalablement à la phase de transfert, en :
   - désactivant le champ magnétique ;
   - libérant les particules capturées en appliquant depuis un puits contenant une solution de lavage au moins un cycle d'aspiration et de refoulement de la solution de lavage dans le cône de pipette ;
   - appliquant une deuxième phase de capture sur une paroi interne du cône de pipette :
   - appliquant le premier champ magnétique au cône de pipette
appliquant au moins un cycle d'aspiration et de refoulement du mélange contenu dans le cône de pipette dans le puits contenant la solution de lavage.

En d'autres termes, l'invention tire avantage d'un cône de pipette dans lequel des cycles d'aspiration et de refoulement peuvent être réalisés en trempant sa pointe dans un puits. Grâce à de tels cycles, il est possible de capturer la totalité des particules présentes dans un échantillon de volume très supérieur à celui du volume, i.e. le cône, dans lequel l'extraction est réalisée. Il est même possible de faire passer dans le cône un volume cumulé de liquide bien supérieur au volume de l'échantillon lui-même, en réglant le nombre de cycles d'aspiration et de refoulement. De la même manière, le volume de la ou des solution(s) de lavage utilisée(s) le cas échéant lors de l'extraction peut être très supérieur au volume du cône. La phase de migration permet quant à elle de localiser les particules magnétiques dans une portion du cône, la pointe, qui peut tremper dans un puits de volume très réduit. Le volume de la solution de récupération peut donc être faible si nécessaire. Le volume de la solution de récupération est ainsi indépendant du volume de l'échantillon et du volume du cône de pipette dans lequel est réalisée l'extraction. Grâce à l'invention, il est par conséquent possible d'optimiser chaque volume de liquide utilisé, et ainsi optimiser l'extraction.

De plus, en raison de la géométrie des cônes en forme de tube et des cycles d'aspiration refoulement, il est obtenu un brassage efficace de l'échantillon dans les cônes, brassage par exemple mis en œuvre avant la capture, ainsi qu'un lavage efficace, et ce sans faire appel à des mécanismes de type aimants mobiles. En outre, les inventeurs ont noté qu'un lavage efficace est obtenu dans le cône alors même que les particules sont capturées sur la paroi du cône de pipette. Un grand volume de solution de lavage peut être utilisé, augmentant encore l'efficacité du lavage. De fait, toutes les étapes de l'extraction (brassage, capture, lavage, transfert dans une solution de récupération) peuvent être réalisées dans le cône de pipette.

Selon un mode de réalisation, le déplacement du premier champ magnétique consiste à déplacer le cône de pipette parallèlement à un axe longitudinal dudit cône, et à conserver constant le premier champ magnétique, l'axe longitudinal du cône de pipette restant à égale distance du premier champ magnétique lors du déplacement du cône de pipette. En d'autres termes, la migration des particules peut être mise en œuvre de manière simple en déplaçant le cône de pipette par rapport, par exemple, un aimant permanent.

Selon un mode de réalisation, la phase de transfert comprend :
- le placement de la pointe du cône de pipette dans le puits de récupération ;
- et l'application d'un second champ magnétique depuis le fond du puits de récupération de manière à faire migrer dans le puits de récupération les particules magnétiques contenues dans la pointe du cône de pipette.

En particulier, le second champ magnétique est produit par un aimant positionné partiellement ou entièrement sous la pointe du cône de pipette. Le premier champ magnétique appliqué au cône de pipette est désactivé lors de l'application du second champ magnétique.

En d'autres termes, le second champ magnétique permet d'attirer simplement les particules dans le puits de récupération, ce qui augmente la vitesse de récupération des particules magnétiques dans le puits de récupération, ainsi que le nombre de particules récupérées. De plus, le second champ magnétique capture automatiquement les particules magnétiques dans le puits de récupération. Par exemple, si la solution de récupération est un éluant, les composants liés aux particules ont été libérés et un technicien peut pipeter directement la solution qui est dépourvue de particules magnétiques.

Selon une variante privilégiée, la phase de transfert comporte la désactivation du premier champ magnétique suivie de l'application de cycles d'aspiration et de refoulement de la solution du puits de récupération dans la pointe du cône de pipette, ladite application comprenant :
- une première phase d'application des cycles à une première fréquence ;
- suivie d'une deuxième phase d'application des cycles à une deuxième fréquence, inférieure à la première fréquence.

La première phase permet de désagréger efficacement l'amas de particules capturées sur le cône de pipette, également appelé « culot », et ainsi de remettre en suspension les particules dans la solution de récupération. La deuxième phase permet de continuer à brasser la solution tout en ne s'opposant pas à la migration des particules sous l'effet du champ magnétique. Ceci permet d'augmenter encore plus la vitesse de récupération et le nombre de particules récupérées dans le puits de récupération. De plus si la solution est un éluant, dont la fonction est de libérer les composants capturés par les particules magnétiques, ces cycles ont pour effet de brasser les particules dans Γ éluant, ce qui augmente l'efficacité de Γ éluant, en particulier lors de l'utilisation d'une solution d'élution dans le décrochage des analytes des particules magnétiques sans étape de chauffage.

Selon un mode de réalisation, le procédé comprend, préalablement à la phase de capture, une phase de brassage du mélange contenu dans le cône de pipette par l'application d'au moins un cycle d'aspiration et de refoulement dudit mélange dans le cône de pipette. En raison de la géométrie du cône, de forme tubulaire, il est possible d'obtenir un grand débit volumique rapporté à la section du cône, et par conséquent un brassage efficace. De plus, il existe de grandes turbulences dans le cône naturellement générées par l'écoulement du liquide, turbulences qui augmentent l'efficacité du brassage. Avantageusement, un accessoire jetable est prévu dans le cône pour accroître cet effet.

Selon un mode de réalisation, le procédé comprend, préalablement à la phase de transfert, au moins une phase de lavage des particules capturées sur la paroi interne du cône de pipette en :
- en désactivant le champ magnétique ;
- en libérant les particules capturées en appliquant depuis un puits contenant une solution de lavage au moins un cycle d'aspiration et de refoulement de la solution de lavage dans le cône de pipette ;
- en appliquant une deuxième phase de capture sur une paroi interne du cône de pipette :
   ∘ en appliquant le premier champ magnétique au cône de pipette
   ∘ et en appliquant au moins un cycle d'aspiration et de refoulement du mélange contenu dans le cône de pipette dans le puits contenant la solution de lavage.

Selon un mode de réalisation, la libération des particules capturées comprend une phase d'application des cycles de manière à réaliser un va-et-vient d'un ménisque de ladite solution sur un culot de particules capturées dans le cône de pipette, ledit va-et-vient dudit ménisque étant réalisé sur une portion du cône inférieure à la longueur totale du cône de pipette.

Plus particulièrement, la libération des particules capturées comprend une deuxième phase d'application des cycles de manière à aspirer et refouler totalement la solution de lavage du cône. La fréquence d'application des cycles de la deuxième phase est inférieure à la fréquence d'application des cycles de la première phase.

Notamment, préalablement à la libération des particules capturées, le procédé comprend au moins deux phases de lavage mises en œuvre dans deux solutions de lavage distinctes.

Ce mode de réalisation est particulièrement avantageux lorsque les composants contenus dans l'échantillon biologique sont des acides nucléiques (e.g. ADN, ARN). Selon un autre mode de réalisation, les composants contenus dans l'échantillon biologique sont microorganismes (e.g. bactéries, champignons, levures), et dans lequel le procédé comprend une unique phase de capture et une unique phase de lavage.

En particulier, le mélange de l'échantillon avec les particules magnétiques a un volume supérieur à 1 millilitre, et de préférence supérieur ou égal à 2 millilitres, et dans lequel le volume du puits de récupération est inférieur ou égal à 200 micro litres, et de préférence inférieur ou égal à 100 microlitres.

Selon un mode de réalisation, le procédé comprend, préalablement à la phase de transfert, au moins une phase de lavage des particules capturées sur la paroi interne du cône de pipette en :
- en aspirant de la solution de lavage dans ledit cône de pipette ;
- puis en modulant le premier champ magnétique appliqué aux particules magnétiques pour capturer celles-ci sur la paroi interne du cône de pipette ;
- puis en refoulant le liquide de lavage du cône de pipette.

En d'autres termes, la modulation du champ magnétique induit une réorganisation du culot de particules capturé sur la paroi du cône. Notamment, le culot peut changer de forme, s'étaler, glisser ou encore « rouler » sur la paroi du cône de pipette. Cette réorganisation du culot permet d'augmenter encore l'efficacité du lavage, et ce d'autant plus que cette réorganisation peut être mise en œuvre conjointement avec des cycles d'aspiration et de refoulement de la solution de lavage.

En particulier, la modulation du premier champ magnétique est réalisée :
- en déplaçant le cône de pipette parallèlement à un axe longitudinal dudit cône, et en conservant constant le premier champ magnétique ;
- et/ou en faisant défiler des aimants espacés les uns des autres devant les particules capturées.

En d'autres termes, la modulation est obtenue simplement, par exemple par un technicien qui fait glisser une barrette d'aimants ou par un automate qui met un mécanisme simple de translation d'une barrette d'aimants.

Selon un mode de réalisation, le volume du cône de pipette est au moins dix fois supérieur au volume du puits de récupération. Selon un mode de réalisation, le volume du mélange est au moins trois fois supérieur au volume du cône de pipette.

Selon un mode de réalisation, les composants appartiennent au groupe formé des acides nucléiques simple brin ou double brin (ADN et/ou ARN), des microorganismes, des protéines, et des peptides. Les composants sont constitués de tout autres types de molécules en fonction de la fonctionnalisation données aux particules magnétiques.

Le but de la présente invention est également de proposer un dispositif pour la mise en œuvre du procédé venant d'être décrit, qui soit peu encombrant et simple d'utilisation par un technicien de laboratoire.

Selon l'invention, il est décrit un porte-pipette comprenant :
- un socle;
- un évidement formé dans le socle apte à loger de manière amovible un support de puits ;
- un support de pipette comprenant un premier logement dans lequel est apte à être insérée, avantageusement de manière amovible, une pipette équipée d'au moins un cône de pipette tubulaire comprenant une pointe destinée au pipetage de liquide, le premier logement étant ouvert sur l'évidement du socle, le support de pipette étant mobile en translation par rapport au socle selon une direction parallèle à un axe des cônes de pipette et mobile entre une première position dans laquelle la pointe de chaque cône de pipette est logée dans un puits du support de puits et au moins une deuxième position dans laquelle ladite pointe est en dehors dudit puits;
- un second logement apte à loger de manière amovible une pièce aimantée, le second logement faisant face à chacun des cônes de pipette dans une position au-dessus de la pointe de celui-ci lorsque le support de pipette est dans la première position, et le second logement fait face à la pointe du cône de pipette lorsque le support de pipette est dans la deuxième position.

En d'autres termes, le porte-pipette reçoit une pipette et le technicien met en œuvre les étapes du procédé d'extraction, en montant/descendant la pipette, notamment pour faire migrer le culot de particule dans la pointe du ou des cônes de pipette, en introduisant des puits (sous la forme de plaque, de barrette, etc) dans le socle, et en actionnant la pipette.

Le porte-pipette, qui est peu encombrant et transportable, permet en outre la semi-automatisation du procédé d'extraction lorsqu'une pipette électronique est utilisée. Une telle pipette comprend en effet des circuits d'aspiration et de refoulement dans chaque cône de pipette l'équipant, et un circuit électronique à base de microprocesseur. Ce circuit électronique pilote les circuits d'aspiration/refoulement en fonction de consignes entrées par le technicien au moyen d'une interface équipant la pipette et/ou d'un ordinateur/tablette/smartphone connecté à la pipette (e.g. par une liaison sans fil de type bluetooth), etc. Ces consignes sont par exemple constituées d'instructions de cycles d'aspiration/refoulement et/ou d'un choix d'un protocole particulier préenregistré dans la pipette.

La pipette électronique étant programmable, une grande polyvalence est en outre obtenue dans la définition du procédé d'extraction, qui peut être adapté en fonction d'une capture magnétique particulière souhaitée (e.g. : purification d'acides nucléiques, immuno-concentration magnétiques,...). Notamment, un protocole approprié à l'extraction visée peut être enregistré dans la pipette, le protocole étant défini en termes de nombre de cycles d'aspiration et de refoulement, d'enchaînement de cycles, de fréquence de cycles, de durée entre les cycles, volumes définis, etc. Un système autonome et semi-automatisé est ainsi obtenu.

Enfin, les cônes de pipette sont détachables de la pipette, et donc aisément remplaçables, sans que la pipette ne soit mise hors service pendant une longue durée.

Selon un mode de réalisation, le premier logement comprend une ouverture pour l'insertion et le retrait frontaux de la pipette dans le premier logement du support de pipette. L'insertion et le retrait frontaux de la pipette et des cônes en position minimisent le risque de toucher le porte-pipette avec les pointes des cônes, et donc le risque de contamination du porte-pipette.

Selon un mode de réalisation, le second logement est réalisé dans le socle.

En particulier, le support de pipette comporte un troisième logement dans lequel la pièce aimantée est apte à être logée de manière amovible pour faire face à chaque cône de pipette à une position au-dessus de la pointe dudit cône lorsque le support de pipette est dans la deuxième position.

En d'autres termes, lorsque la pièce aimantée (e.g. comprenant un ou plusieurs aimants permanents) est présente dans le troisième logement, elle est solidaire des cônes de pipette et suit donc leur mouvement de translation par rapport au socle. Lors de tels mouvements, la pièce aimantée conserve donc les culots de particules magnétiques fixes dans les cônes. Le technicien peut ainsi par exemple monter la pipette pour déplacer plus aisément un support à puits dans le socle sans risquer de déplacer les culots de particules dans les cônes.

Selon un mode particulier, le second et troisième logements communiquent, et le support de pipette comprend des moyens aptes à maintenir de manière amovible la pièce aimantée dans le troisième logement. De cette manière, le technicien peut détacher la pièce aimantée du support à pipette qui prend alors place automatiquement dans le socle en tombant dans le deuxième logement. Ce détachement a notamment lieu pour l'opération de migration des particules magnétiques dans les pointes des cônes.

Selon un mode de réalisation :
- le socle comprend au moins une roue dentée mobile en rotation ;
- et le support de pipette comprend une crémaillère coopérant avec la roue dentée pour translater le support de pipette par rapport au socle lors de la rotation de la roue dentée.

En particulier, le porte-pipette comprend un dispositif de verrouillage et de déverrouillage du support de pipette dans la première position. Notamment, le porte-pipette comprend au moins une poignée solidaire de la roue dentée pour faire tourner celle-ci et apte à se fixer de manière amovible à une poignée solidaire de la roue dentée d'un autre porte-pipette, ce qui permet donc d'augmenter le nombre de cônes de pipette pendant le procédé d'extraction.

Selon l'invention, il est décrit un système pour l'extraction de composants contenus dans un échantillon biologique sous forme liquide, lesdits composants étant aptes à se fixer sur des particules magnétiques, le système comprenant :
- une pipette équipée d'au moins un cône de pipette tubulaire comprenant une pointe destinée au pipetage de liquide et d'un circuit d'aspiration et de refoulement dans chaque cône de pipette ;
- au moins un support de puits ;
- un porte-pipette comprenant :
   o un socle ;
   o un évidement formé dans le socle apte à recevoir de manière amovible chaque support de puits ;
   o un support de pipette comprenant un premier logement dans lequel la pipette est insérée, avantageusement de manière amovible, le premier logement étant ouvert sur l'évidement du socle, le support de pipette étant mobile en translation par rapport au socle selon une direction parallèle à un axe des cônes de pipette et mobile entre une première position dans laquelle la pointe de chaque cône de pipette est logée dans un puits du support de puits et au moins une deuxième position dans laquelle ladite pointe est en dehors dudit puits;
   o un second logement faisant face à chacun des cônes de pipette dans une position au- dessus de la pointe de celui-ci lorsque le support de pipette est dans la première position et le second logement faisant face à la pointe du cône de pipette lorsque le support de pipette est dans la deuxième position ; et
      - une pièce aimantée logée de manière amovible dans le second logement.

Notamment, le porte-pipette est conforme au porte-pipette décrit plus haut.

Selon l'invention, il est décrit un support de puits pour la migration de particules magnétiques depuis des pointes de cônes de pipettes dans des puits de récupération.

A cet effet, l'invention a également pour objet un support de puits, comprenant une pièce dans laquelle sont formés des évidements pour la réception des puits, et au moins un aimant faisant face à chacun des évidements formés dans ladite pièce.

### BRÈVE DESCRIPTION DES FIGURES

L'invention sera mieux comprise à la lecture de la description qui va suivre, donnée uniquement à titre d'exemple, et faite en relation avec les dessins annexés, dans lesquels des références identiques désignent des éléments identiques, et dans lesquels :
- la figure 1 est une vue en perspective d'un système d'extraction permettant la mise en œuvre du procédé d'extraction selon l'invention ;
- les figures 2A et 2B sont des vues de face et en perspective d'une pipette électronique et de ses cônes de pipettes amovibles ;
- les figures 3A et 3B est des vues en perspective et de face d'un porte-pipette permettant la mise en œuvre du procédé d'extraction selon l'invention ;
- les figures 4A et 4B est une vue de détail en coupe du porte-pipette de la figure 3, respectivement selon les plans A-A et B-B de la figure 3B ;
- la figure 5 est une vue en perspective d'une plaque « deepwell » comprenant des puits ; les figures 6A et 6B sont des vues en perspective d'un portoir magnétique et de tubes d'élution PCR pouvant se loger dans le portoir ;
- la figure 7 est une vue de face d'une pièce aimantée;
- la figure 8 est un organigramme d'un procédé d'extraction selon l'invention ;
- la figure 9 est une photographie des cônes de pipette d'un système permettant la mise en œuvre du procédé d'extraction selon l'invention avec des culots de particules magnétiques placés environ à mi-hauteur des cônes ;
- la figure 10 est une photographie de ces mêmes cônes avec les culots placés dans les pointes de ceux-ci ;
- la figure 11 est une photographie de tubes d'élution PCR dans lesquels ont été récupérées les particules magnétiques :
- les figures 12 et 13 illustrent un second mode de réalisation du porte-pipette permettant la mise en œuvre du procédé d'extraction selon l'invention ;
- la figure 14 est une vue en perspective de deux systèmes selon la figure 1 couplés au moyen de poignées de rotation ;
- la figure 15 est une vue schématique illustrant le va-et-vient d'un ménisque d'une solution sur un culot de particules pour détacher ce dernier de la paroi d'un cône de pipette .

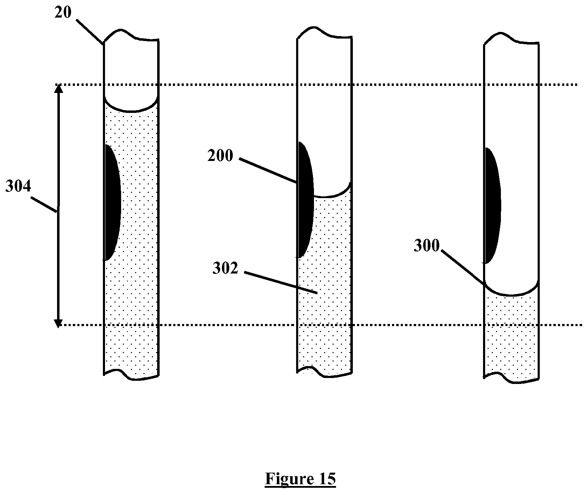

Excepté pour la figure 15, la description est réalisée en relation avec des plans et photographies à une échelle réduite d'un système réel.

### DESCRIPTION DÉTAILLÉE DE L'INVENTION

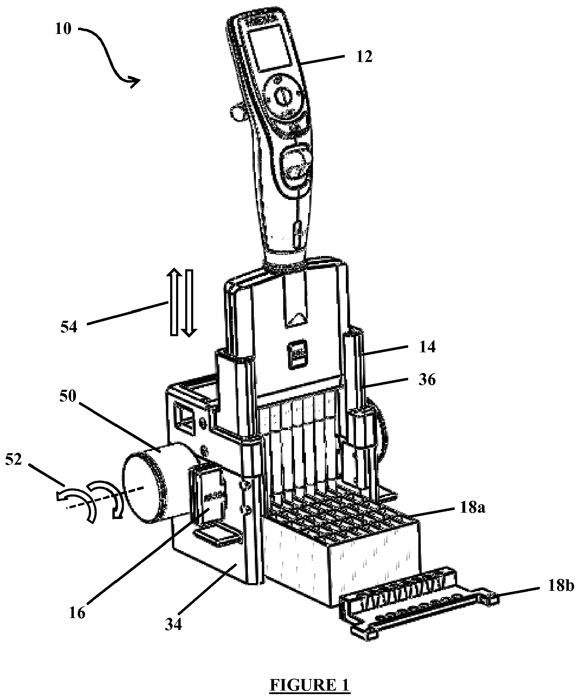

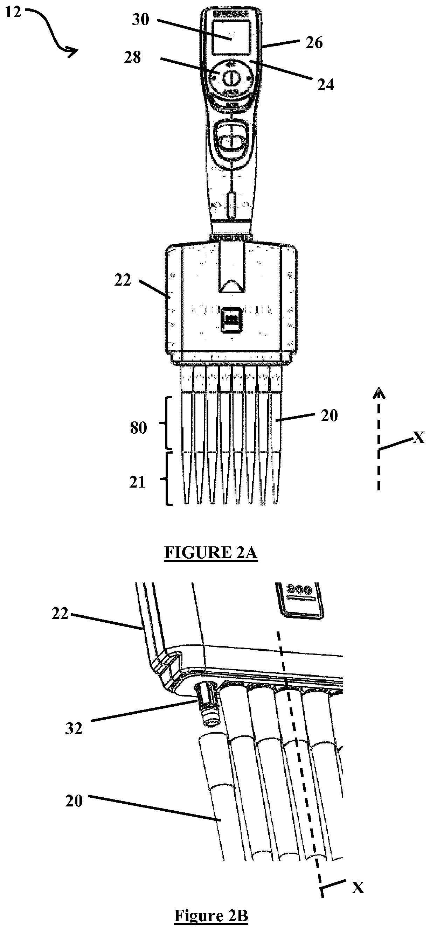

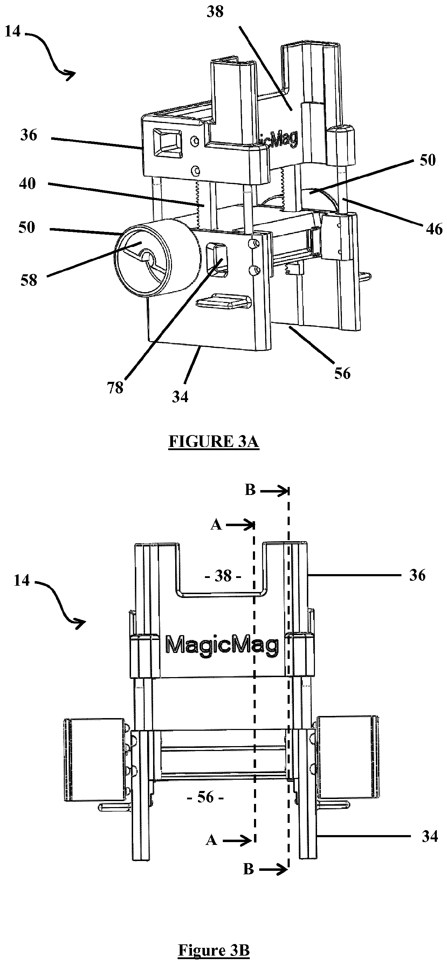

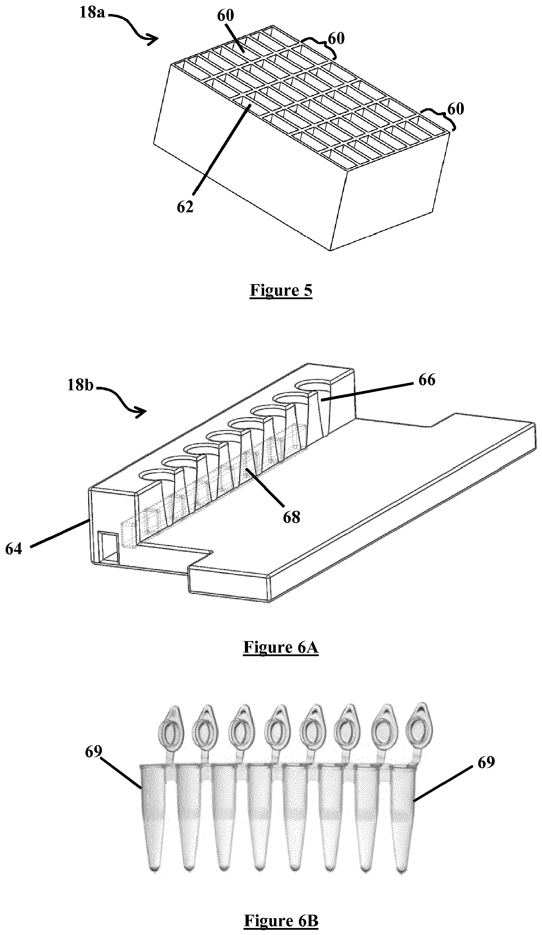

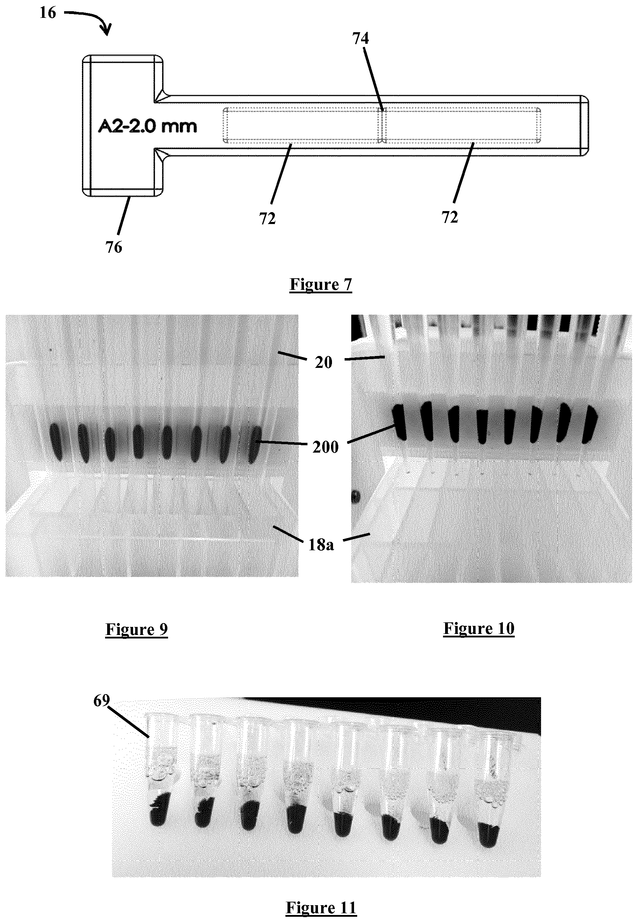

En se référant aux figures 1 à 7, un système 10 d'extraction (figure 1) de composants contenus dans un échantillon liquide comporte une pipette électronique 12 (figures 2), un porte-pipette 14 (figures 3) dans lequel la pipette 12 est logée, un ou plusieurs support de puits 18a, 18b (figure 5 et 6) pouvant chacun être logé dans le porte-pipette 14, et une première pièce aimantée 16 (figure 7).

La pipette 12, qui est portable, comprend une rangée de cônes de pipettes 20, et un corps 22 sur lequel sont montés les cônes 20 (figure 2A). Dans ce corps 20, sont logés un circuit d'aspiration/refoulement de liquide dans les cônes 20 (par exemple un ensemble de pistons actionnés par un moteur électrique), et un circuit électronique de pilotage du circuit d'aspiration/refoulement. Le circuit électronique, qui comprend par exemple un microprocesseur et une ou plusieurs mémoires informatiques, est programmable, et embarque des instructions pour la mise en œuvre d'un ou plusieurs protocoles de pipetage, chaque protocole comprenant une ou plusieurs étapes. Le circuit électronique comprend également une interface homme machine 24 logée dans une poignée 26 du corps 22, l'interface comprenant un ensemble de boutons de sélection et de navigation 28 et un écran d'affichage 30 permettant la visualisation et la sélection de différents protocoles de pipetage enregistrés. L'utilisateur peut notamment programmer la pipette électronique 12, e.g. en téléchargeant dans celle-ci des instructions depuis un ordinateur connecté à la pipette 12 au travers d'une liaison sans fil, par exemple Bluetooth. L'utilisateur peut également sélectionner, via l'interface 24, un protocole préenregistré. La pipette 12 est par ailleurs apte à aspirer un volume prédéfini de liquide dans chacun des cônes 20, refouler un volume prédéfini depuis chacun des cônes, mettre en œuvre des cycles automatiques d'aspiration/refoulement de durée et de fréquence variable.

Comme illustré plus particulièrement à la figure 2B, les cônes 20, de forme tubulaires et d'axe longitudinal X, sont amovibles en s'emboîtant dans des lobes 32 faisant saillie du corps 22, ce qui permet leur remplacement. Les cônes sont par ailleurs constitués d'un matériau plastique, par exemple en polypropylène, ce qui a pour effet de les rendre « transparent » à un champ magnétique et permet la capture de particules magnétiques, comme cela sera décrit plus en détails par la suite. Enfin, chaque cône 20 présente à son extrémité ouverte de pipetage un profil effilé 21, ou « pointe ». Cette partie 21 a une section réduite dans un plan perpendiculaire à l'axe X, ce qui permet son introduction facilitée dans des récipients ou puits comme cela est connu en soi.

La pipette électronique 12 est par exemple le modèle « VIAFLO II 8 canaux » commercialisé par la société ©INTEGRA Biosciences AG, Suisse, modèle dont les éléments sont décrits dans les demandes de brevets US 2009/071266, US 2009/074622, US2011076205 et US 2008/095671.

Le porte-pipette 14 comporte quant à lui (figures 3A et 3B) un socle 34, destiné à être posé sur un plan de travail (e.g. une table ou une paillasse de laboratoire), ainsi qu'une partie mobile 36 par rapport au socle 34. La partie mobile 36, également nommée « support de pipette », comporte un logement 38 dans lequel la pipette 12 peut être insérée de manière amovible et maintenue immobile comme illustré à la figure 1. Pour la translation du support de pipette 36 par rapport au socle 34, le support 36 comporte une ou plusieurs crémaillères 40, par exemple au nombre de deux, s'engrenant avec des roues dentées 42 montées sur un arbre 44 mobile en rotation et logé dans le socle 34. Une ou plusieurs tiges 46 sont en outre fixées au support 36 (resp. dans le socle 34) et coulissent dans des orifices du socle 34 (resp. dans le support 36), afin de guider le support 36 dans son mouvement de translation. Une ou deux poignées de rotation 50 sont en outre fixées au bout de l'arbre 44 afin de permettre à l'utilisateur de facilement tourner ce dernier selon les flèches 52 (figure 1) et donc faire monter et descendre le support de pipette 36 tel qu'illustré par les flèches 54 (figure 1). Le mouvement de translation du support de pipette 36 par rapport au socle 34 est donc parallèle à l'axe X des cônes 20 lorsque la pipette 12 est placée dans le logement 38 du support 36, et donc parallèle à la direction de la gravité lorsque le socle 34 est posé sur un plan de travail horizontal, de sorte que le support 36 « monte » ou « descend».

Le socle 34 est ouvert sur sa face avant 56 pour permettre l'introduction et le retrait des supports de puits 18a, 18b, définissant ainsi un logement pour ces derniers. Ce logement est ouvert sur sa partie supérieure pour permettre aux cônes 20 de la pipette 12 d'atteindre lesdits supports de puits lorsque la pipette descend. Ainsi, comme décrit plus en détail ci-après, la pipette 12 peut prendre plusieurs positions par rapport au socle 34, et donc par rapport à un support de puits 18a, 18b logé dans ce dernier. En particulier, la pipette 12 peut prendre une position dans laquelle les pointes 21 des cônes 20 trempent dans des puits du support 18a, 18b, et au moins une position dans laquelle les pointes 21 ne trempent pas dans les puits, et sont à distance de ces derniers de manière à permettre la manipulation des supports de puits par l'utilisateur et la capture de particules magnétiques dans une position centrale des cônes 20.

En se référant aux figures 5 et 6, les supports de puits peuvent prendre plusieurs formes en fonction de l'extraction souhaitée. Notamment, un support de puits est une plaque 18a compartimentée (figure 5), usuellement nommée « microplaque » de type « DeepWell ». Ce type de plaque comporte des rangées 60 de puits 52 dans lesquelles peut plonger la rangée de cônes de pipette 20. Chaque rangée 60 peut ainsi recevoir un liquide particulier utilisé lors de l'étape d'extraction mise en œuvre par le système 10. Le passage d'une rangée 60 à l'autre est alors réalisé simplement par l'utilisateur qui met à l'aplomb des cônes de pipettes 20 la rangée 60 particulière contenant le liquide nécessaire à l'étape devant être mise en œuvre.

Un autre support de puits, décrit à la figure 6A, est aimanté et spécifiquement conçu pour la migration de particules magnétiques depuis les pointes 21 des cônes de pipettes 20 dans des puits. Le support 18b comprend à cet effet un corps 64 dans lequel est formée une rangée de de logement 66 pouvant recevoir la rangée de cône de pipette 20, et dans lequel est logée une seconde pièce aimantée 68 comprenant un ou plusieurs aimants permanents, par exemple un aimant permanent à proximité de chacun des puits 66. La pièce aimantée 68 est placée sous les puits 66 ou en face de leur portions inférieures comme illustré. Ainsi, les pointes 21 des cônes de pipettes 20 sont placées au-dessus de la pièce aimantée 68 lorsque lesdites pointes sont plongées dans les puits 66. Enfin, le support 18b sert de portoir magnétique revevant de manière amovible des tubes 69 dans les logements 66, par exemple des tubes d'élution PCR tels qu'illustrés à la figure 6B, à des fins par exemple de transfert ultérieur du produit de l'extraction récupéré dans ceux-ci.

La première pièce aimantée 16, dont la fonction est de capturer des particules aimantées dans les cônes 20 d'une manière décrite plus en détail par la suite, comprend quant à elle un ou plusieurs aimants permanents 72, avantageusement une rangée d'aimants permanents séparés les uns des autres par des espaces 74, et encore plus avantageusement un aimant permanent en face de chaque cône de pipette 20 lorsque la pièce 16 est entièrement logée dans le socle 34. La pièce 16 comprend en outre une poignée 76 pour une meilleure préhension par l'utilisateur.

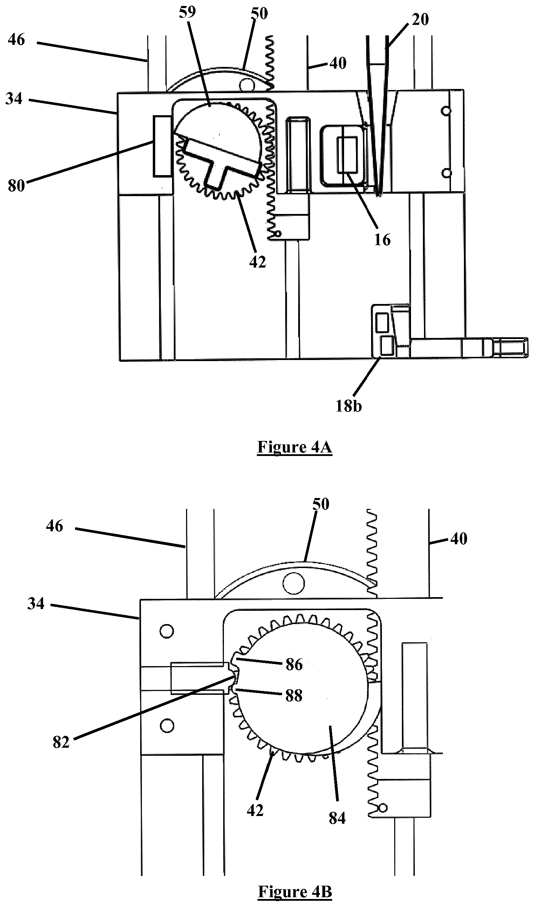

Un logement 78 pour la réception de la pièce aimantée 16 est prévu dans le socle 34, le logement 78 étant placé de manière à ce que la pièce 16 soit en face des cônes de pipette 20 au-dessus de leur pointe 21, et de préférence face à une zone centrale 80 à une hauteur supérieure au puits, lorsque les pointes 21 plongent dans les puits maintenu dans un support de puits. De cette manière, les particules sont capturées dans un volume du cône suffisamment grand pour ne pas former de bouchons dans les cônes. Le porte-pipette 14 comporte également des moyens permettant de maîtriser la vitesse de remontée du support 36. En particulier, la crémaillère et la roue dentée sont conçus pour qu'un demi-tour (180°) de la roue 50 permette de parcourir l'ensemble de la crémaillère, et une masselotte 58 intégrée dans chacune des poignées 50 de manière désaxée par rapport à l'arbre 44. Ces masselottes, sous leur poids et l'effet de levier associé, génèrent un couple de rotation mettant en rotation l'arbre 44 tout en limitant le couple transmis à la main par l'utilisateur. De manière avantageuse, comme illustré à la figure 4A, une partie substantielle de l'arbre 44 est formé également d'une masselotte demi- cylindrique dans le même but. Cette assistance mécanique aide à soulever le support-de pipette, et limite donc les troubles musculo-squelettiques, et met en œuvre un frein qui permet à l'utilisateur de contrôler avec plus de précision la vitesse de montée et de descente du support 36.

D'autres mécanismes de contrôle de la vitesse du support 36 peuvent être prévus, notamment un freinage magnétique. Par exemple, en se référant à la figure 4A, la masselotte 59 comprend un matériau aimantable (e.g. acier ou équivalent) et une troisième pièce aimantée 80 (parallélépipédique ou en forme d'arc de cercle concentrique à l'arbre 44) est logée dans le socle 34, de préférence à l'opposé de la pièce aimantée 16 vis-à-vis de l'arbre 44 pour ne pas perturber l'extraction. Lorsque la masselotte 59 de l'arbre passe devant la pièce aimantée 80, le mouvement de rotation de l'arbre 44 est ralenti en raison du couple de freinage engendré. Ceci permet d'une part de compenser l'effort pour soulever la pipette lors de la montée (en jouant le rôle d'assistance pour l'utilisateur), et d'autre part de maîtriser la vitesse de remontée de la pipette lorsque l'on veut faire descendre les particules magnétiques en bas des cônes de pipette, comme cela sera décrit ci-dessous.

Un mécanisme de butée est également avantageusement prévu comme illustré à la figure 4B. Dans cette variante, une roue 84 en matériau déformable (e.g. en élastomère) est montée sur l'arbre 44 et comporte deux dents 86, 88. Une protubérance 82, par exemple hémisphérique, fait par ailleurs saillie du socle 84 en face de la roue 84. Lorsque l'utilisateur remonte la pipette 12 en actionnant la roue 50, la première dent 86 rencontre la protubérance 82. En augmentant le coupe appliqué à la roue 50, la dent 86 se plie, passe la protubérance 82, et reprend sa forme. Dans cette position, la dent 86 peut alors reposer sur la protubérance 82, la dureté de cette dent étant choisi pour qu'elle ne se plie pas sous l'action du poids de la pipette 12 et du porte-pipette 36. La pipette est ainsi bloquée en position haute, l'utilisateur pouvant donc relâcher la roue 50. La deuxième dent 88, de plus grande dimension (e.g. longueur et/ou largeur) nécessite pour son passage un couple beaucoup important, et définit ainsi une butée pour éviter que le porte-pipette 36 ne se déboite du socle 34, à moins que l'utilisateur déploie une force apte à casser cette dent. En variante, la protubérance 82 est remplacée par une butée comportant une bille montée sur ressort dans un logement du socle. La roue 84 peut ainsi être réalisée un matériau dur. L'action de la première dent 86 a alors pour effet de pousser la bille dans son logement, permettant ainsi le passage de la dent 86.

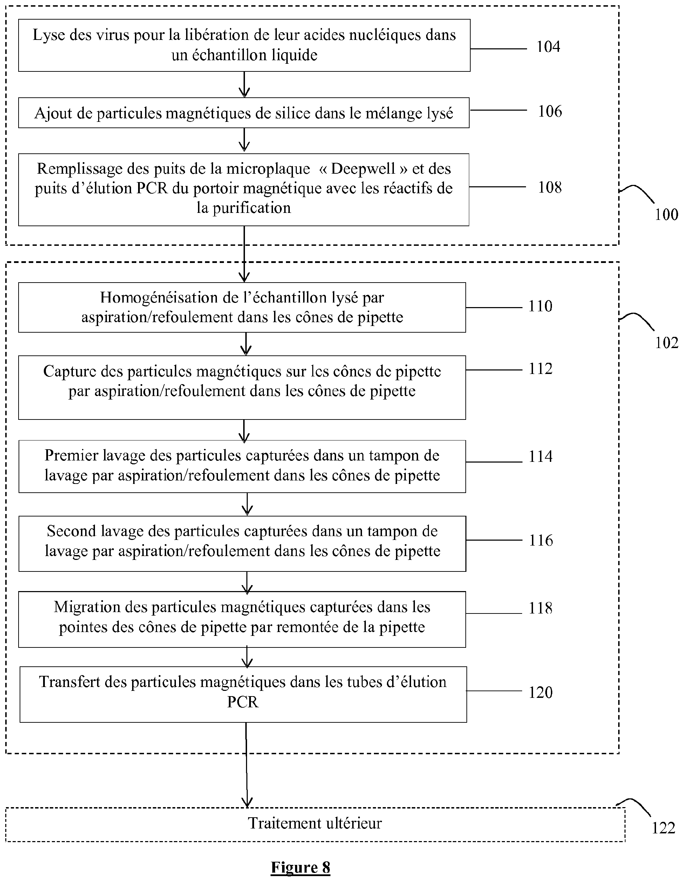

Il est à présent décrit un procédé d'extraction de composants contenus dans un échantillon liquide à l'aide de particules magnétiques, ce procédé étant mis en œuvre à l'aide du système venant d'être décrit. Le procédé se fonde sur l'association du porte-pipette, de la pipette électronique programmable et de cônes de pipette (e.g. d'un volume de 1250 µí) afin de réaliser les différentes étapes de capture, de lavage et d'élution de particules magnétiques pour traiter un volume d'échantillon par cône de pipette compris entre 1 mL et 5 mL. La capture des particules magnétiques s'effectue séquentiellement dans les cônes de pipette au cours de cycles d'aspiration/refoulement sur l'ensemble du volume d'échantillon à traiter. A titre d'exemple, il est décrit en relation avec l'organigramme de la figure 8 un procédé de purification d'acides nucléiques viraux utilisant la chimie NucliSENS©, à savoir une extraction des acides nucléiques à l'aide de particules de silice magnétiques.

Le procédé débute par une étape 100 de préparation des différents échantillons et réactifs nécessaires à la purification, suivie de ladite purification en 102.

Notamment, la préparation 100 consiste, en 104, à mélanger l'échantillon biologique comprenant des virus dont on souhaite extraire les acides nucléiques, avec un réactif de lyse chimique des virus (e.g. le réactif de lyse « Nuclisens miniMAG » de bioMérieux, de référence 200292, ou le réactif de lyse « Nuclisens easyMAG » de bioMérieux, de référence 280130), à raison de deux volumes de réactif de lyse pour un volume d'échantillon. Le mélange est ensuite chauffé pendant 30 minutes à 56°C, libérant ainsi les acides nucléiques des virus d'une manière connue en soi. Des particules de silice magnétiques (e.g. des particules ayant un cœur paramagnétique, ferromagnétique ou ferrimagnétique présentant ou non une rémanence, recouvert d'une coque en silice), ayant pour propriété de se lier avec des acides nucléiques, sont alors introduites, en 106, dans l'échantillon lysé.

La préparation 100 se poursuit, en 108, par le remplissage de la microplaque 18a, ayant des puits 62 de 5mL, et des tubes d'élution PCR 69 de 0,2 mL du portoir magnétique 18b de sorte que :
- chaque puits de la première rangée de la microplaque 18a est rempli de l'échantillon lysé comprenant les particules de silice, ci-après « échantillon lysé ». Le volume total dans chaque puits de la première rangée est de préférence supérieur à 1,5 mL en raison de l'utilisation de la microplaque Deepwell 5 mL et des volumes manipulés par la pipette électronique; chaque puits 66 de la deuxième rangée de la microplaque 18a est rempli de 1250 µ 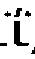 , de tampon de lavage (e.g. le « NucliSENS easyMAG Extraction Buffer n°2 » de bioMérieux de référence bMx 280131) ;
- chaque puits 66 de la troisième rangée de la microplaque 18a est rempli de 1250 µ 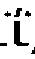 , de tampon de lavage (e.g. le « NucliSENS easyMAG Extraction Buffer n°2 » de bioMérieux de référence bMx 280131) ;
- chaque tube d'élution PCR 69 logé dans le portoir magnétique 18b est rempli d'un volume de 100 de tampon d'élution (e.g. le « NucliSENS easyMAG Extraction Buffer n°3 » de bioMérieux, de référence 280132).

L'utilisateur place alors :
- la pipette électronique 12, avec sa rangée de cônes 20, dans le logement 38 du porte-pipette 14, en position relevée pour permettre l'introduction de la plaque 18a ; et
- la plaque 18a dans le logement 56 du socle 34 avec la première rangée de puits comprenant l'échantillon lysé à l'aplomb des cônes 20.

L'extraction 102 débute par l'homogénéisation de l'échantillon lysé. Pour se faire, la pièce aimantée 16 n'est pas placée dans le socle 34 et n'interfère donc pas avec les cônes 20. L'utilisateur tourne une des roues 50 de manière à plonger les pointes 21 des cônes 20 dans la rangée de puits de la plaque 18a comprenant l'échantillon lysé. Puis, il sélectionne à l'aide de l'interface 24 de la pipette 12 un premier protocole de pipetage comprenant au moins une phase d'aspiration/refoulement de l'échantillon lysé dans les cônes 20, et lance le protocole sélectionné. Ces phases (e.g. au nombre de deux) comprennent chacune au moins un cycle d'aspiration/refoulement (e.g. cinq cycles) suivi d'une durée d'attente de plusieurs minutes, par exemple 5 minutes. Au sens de l'invention, un cycle d'aspiration et de refoulement consiste à remplir au moins au trois quart, par exemple complètement, les cônes puis à les vider complètement, à moins qu'il ne soit spécifié autrement par le programme.

Une fois l'homogénéisation terminée, les cônes 20 sont vides et leurs pointes 21 plongent dans les puits contenant l'échantillon lysé. La purification 102 se poursuit par la capture, en 112, des particules de silice de l'échantillon lysé sur la paroi interne des cônes 20. A cet effet, l'utilisateur place la pièce aimantée 16 dans le logement 78 du socle 34, sélectionne à l'aide de l'interface 24 de la pipette 12 un second protocole de pipetage puis lance le protocole sélectionné. Le second protocole comprend une pluralité de cycles d'aspiration/attente/refoulement, e.g. une dizaine de cycles, une aspiration étant séparée d'un refoulement de quelques secondes, e.g. une dizaine de secondes. A chaque aspiration et chaque refoulement, une partie des particules contenues dans l'échantillon lysé est capturée sur la paroi des cônes de pipette grâce au champ magnétique produit par la pièce aimantée 16.

Les particules magnétiques, et donc également leurs acides nucléiques liés, sont ainsi capturées sous le forme de culots de particules 100 en face de la pièce aimantée 16, et de préférence sur une zone centrale à mi-hauteur des cônes 20, comme illustré à la figure 9.

Une fois la capture terminée, l'échantillon lysé ayant été refoulé complètement des cônes 20 et la pièce aimantée 16 étant toujours en place, la purification 102 se poursuit par une première étape de lavage 114. A cette fin, l'utilisateur soulève le porte-pipette 14 (resp. remonte la pipette 12) de manière à libérer la plaque 18a des cônes 20, aligne la deuxième rangée de la plaque 18a avec la rangée de cône 20 puis replace le porte-pipette (resp. fait descendre la pipette) de manière à faire tremper les pointes 21 des cônes dans les puits de la plaque 18a. L'utilisateur sélectionne ensuite, à l'aide de l'interface 24, un troisième protocole de pipetage comprenant au moins une phase d'aspiration/refoulement de l'échantillon lysé dans les cônes 20, puis lance le protocole sélectionné. Le troisième protocole est par exemple identique au premier protocole. Le passage répété du tampon de lavage sur les culots de particules permet ainsi de laver ces derniers. Cette étape de lavage est avantageusement complétée, ou mise en œuvre de manière conjointe, à une modulation du champ magnétique capturant les particules sur les cônes. Par exemple, l'utilisateur fait remonter et descendre la pipette 12, ce qui a pour effet de déplacer les culots de particules sur les cônes, ou bien la pièce aimantée 16 comprend un train d'aimants permanents et l'utilisateur fait coulisser dans un mouvement de va-et-vient la pièce aimantée 16 de son logement 78, de sorte que l'intensité et les lignes de champs magnétiques capturant les culots varient, tout en conservant les particules capturées sur les cônes. La modulation du champ magnétique a ainsi pour effet de réorganiser les culots lors du lavage, et augmenter l'efficacité de celui-ci.

Un second lavage est ensuite mis en œuvre en 116 à l'aide du tampon de lavage de la troisième rangée de la plaque 18a. Par exemple, les cônes sont complètement vidés du premier tampon de lavage, puis une second lavage identique au premier lavage est réalisé.

A la suite de ce second lavage, une étape de migration 118 des culots de particules 200 dans les pointes 21 des cônes 20 est mise en œuvre. Pour ce faire, les cônes 20 restent préférentiellement remplis du second tampon de lavage pour faciliter la glisse des culots 200 et restent alignés avec la seconde rangée de la plaque 18a. L'utilisateur tourne alors une des roues 50 pour faire remonter la pipette 12. La pièce aimantée 16 étant solidaire du socle 34, les culots restent donc immobiles par rapport à cette dernière et migrent vers les pointes 21 en glissant sur les parois des cônes 20 à mesure de la remontée de la pipette. L'utilisateur stoppe la remontée de la pipette 12 une fois les culots 200 dans les pointes 21, comme illustré à la figure 10, à une distance moyenne de quelques millimètres, e.g. 8 mm, des extrémités ouvertes des cônes. Dans cette position, l'utilisateur sélectionne et lance ensuite à l'aide de l'interface 24 le refoulement du tampon de lavage contenu dans les cônes 20 dans les puits de la plaque 18a. Optionnellement, une des phases de lavage, ou une phase de lavage supplémentaire consiste à retirer la pièce aimantée de manière à libérer les particules magnétiques et réaliser un lavage tout en brassant les particules dans la solution de lavage par des cycles d'aspiration et de refoulement. Les particules sont ensuite capturées une nouvelle fois en replaçant la pièce aimantée et en procédant à des cycles d'aspiration et de refoulement tel que décrit précédemment.

La purification 102 se termine par une étape 120 de transfert dans les tubes d'élution PCR 69 des particules magnétiques présentes dans les pointes 21 des cônes 20. A cet effet, l'utilisateur soulève le porte-pipette 14, ôte la plaques 18a, place le portoir magnétique 18b dans le logement 56 de manière à aligner les tubes PCR 69 avec la rangée de cônes 20, repose le porte-pipette 14 et retire la pièce aimantée 16 du socle 14 afin de libérer les particules magnétiques capturées des cônes. Une fois les pointes 21 plongées dans les tubes 69, l'utilisateur sélectionne, à l'aide de l'interface 24, un quatrième protocole de pipetage, puis lance le protocole sélectionné. Une première variante de ce protocole consiste en des cycles d'aspiration et de refoulement du tampon d'élution dans les pointes 21 des cônes 20, ce qui permet la remise en suspension des particules magnétiques en cassant les culots de particules. Par ailleurs, la fréquence choisie pour les cycles permet, à chaque refoulement dans les tubes 69, à une parties des particules magnétiques d'être capturées dans les tubes 69 grâce au champ magnétique de la pièce aimantée 68 logée dans le portoir 64. De plus ces cycles permettent de « rincer » les pointes 21 pour récupérer des particules adhérant aux parois des cônes. Dans une deuxième variante du protocole, des cycles d'aspiration et de refoulement sont tout d'abord mis en œuvre à une fréquence plus élevée de façon à brasser plus vigoureusement le tampon et les particules, et donc d'obtenir une homogénéisation accélérée facilitant le transfert dans les tubes d'élution 69. L'étape de transfert se termine par le refoulement complet du tampon d'élution dans les tubes 69. Sous l'effet du champ magnétique du portoir 64, les particules magnétiques sont alors définitivement séparées du tampon d'élution, comme illustré à la figure 11. L'utilisateur peut ainsi récupérer les tubes 69 pour un traitement ultérieur, en particulier l'élution des acides nucléiques par chauffage, d'une manière connue en soi.

Dans le mode de réalisation du porte-pipette décrit précédemment, la pièce aimantée 16 est logée dans le socle 34. Aussi, lorsque l'utilisateur souhaite avancer la plaque 18a, il peut remonter la pipette suffisamment haut pour procéder à cette opération. Ceci provoque, comme pour la migration des particules vers les pointes, le déplacement des culots 200 sur les parois des cônes 20, ce qui présente l'avantage de « réorganiser » les culots qui peuvent rouler sur eux-mêmes. L'efficacité du lavage s'en trouve ainsi renforcée. Par contre, cela implique que l'utilisateur prend garde à ne jamais trop remonter la pipette afin d'éviter que les culots sortent des cônes. Pour ce faire, l'utilisateur peut par exemple soulever ou basculer le porte pipette pour conserver les culots à distance des ouvertures des cônes. Cette option, qui nécessite le soulèvement répété d'un dispositif dont le poids peut être important, peut cependant entraîner à long terme des troubles musculo-squelettiques. En outre, l'utilisateur doit encore prendre garde de ne pas trop soulever le porte-pipette afin d'éviter que les culots sorte des cônes.

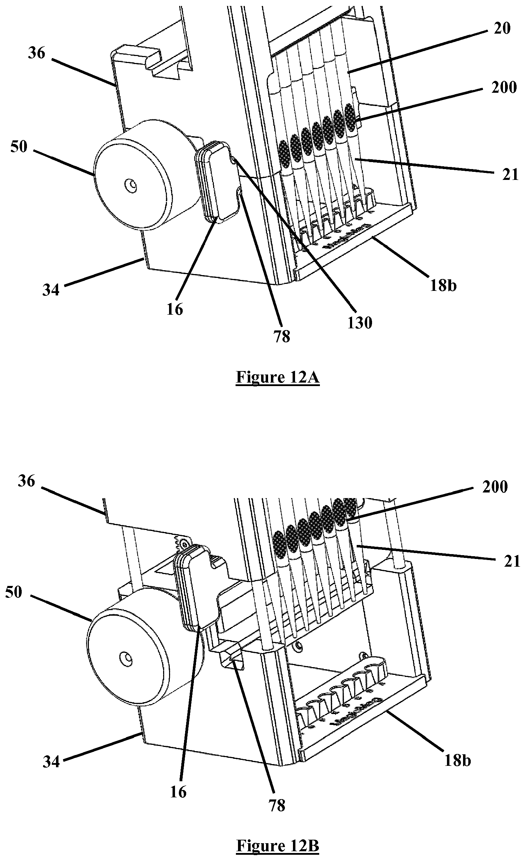

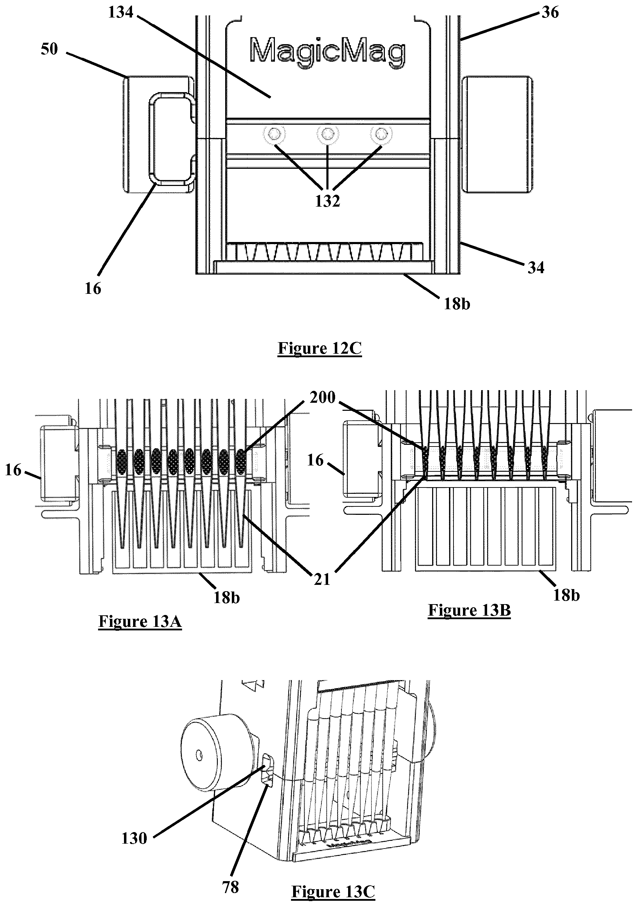

Une second mode de réalisation du porte-pipette selon l'invention permet la manipulation des plaques 18a, 18b en remontant uniquement la pipette, et donc en évitant de soulever le porte- pipette 14, tout en garantissant que les culots de particules restent à distance des pointes 21 des cônes. Ce second mode de réalisation, ainsi que les variations induites sur le procédé venant d'être décrit, sont illustrés aux figures 12 et 13.

Plus particulièrement, le second mode de réalisation diffère du premier mode de réalisation par les moyens de réception de la pièce aimantée 16 dans le porte-pipette 14. Notamment, le socle 34 comprend le logement 78 pour l'insertion et le retrait de la pièce aimantée 16 comme décrit précédemment et le logement 78 est ouvert dans sa partie supérieure 130 pour permettre également l'insertion et le retrait de la pièce 16 verticalement dans le logement 78. Le support de pipette 36 comprend en outre des moyens pour fixer la pièce aimantée 16 à l'aplomb du logement ouvert 78, en particulier un ou plusieurs plots 132 en matériau aimantable (e.g. en acier) fixés sur une paroi arrière 134 du support de pipette mobile 36 (figure 12C). De cette manière, la pièce aimantée 16 est solidaire du support mobile 36, et reste en face des cônes 20 lorsque l'utilisateur remonte et descend la pipette (en particulier lors des phases de lavage), comme illustrée aux figures 12B à 13A.

Pour réaliser la migration des culots 200 dans les pointes des cônes 20, l'utilisateur désolidarise la pièce aimantée 16 du support de pipette 36, en appliquant en simple pression vers le bas sur la poignée 78 de la pièce 16 et remonte la pipette 12. La pièce aimantée 16 se détache des plots 134, reste donc dans le logement 78 du socle et est donc solidaire du socle 34, induisant la migration des culots 200 dans les pointes 21 des cônes comme décrit précédemment (figures 13A et 13B). Une fois la pipette remontée, l'utilisateur remplace la plaque 18a par le portoir 18b muni des tubes d'élution PCR, retire la pièce aimantée 16 et redescente la pipette (figure 13C, tubes 69 non représentés). En variante, le support de pipette 36 peut comporter un logement analogue au logement 78 du socle dans lequel l'utilisateur glisse la pièce aimantée notamment pour les phases de lavage.

Il a été décrit un procédé d'extraction particulier. La présente invention s'applique cependant à tout type de capture de particules magnétiques et à tout type de séquence de pipetage. De même, il a été décrit une pipette ayant 8 canaux d'un volume particulier. La pipette peut comprend un nombre quelconques de canaux de volume quelconque en fonction de l'application visée.

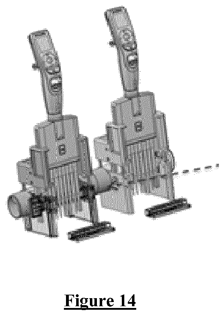

Afin d'augmenter le nombre d'échantillons traités, deux systèmes d'extraction selon l'invention peuvent être couplés, comme illustré à la figure 14. Par exemple, les poignées de rotation 50 peuvent s'emboiter de sorte que deux extractions peuvent être menées simultanément, l'utilisateur remontant et descendant les pipettes 12 en même temps. A cette fin, les pipettes peuvent également être synchronisées, une pipette commandant par exemple l'autre pipette.

De même, il a été décrit un système d'extraction portable et semi-automatisé, particulièrement adapté aux laboratoires d'analyse ayant un nombre limité d'extractions à réaliser au quotidien. L'invention peut cependant être automatisée. Par exemple, la pipette est intégré à un automate qui comprend des mécanismes programmables de montée et de descente de la pipette et de mouvement d'aimant (ou d'activation/désactivation d'électroaimants).

Il a été décrit deux lavages dans les puits de la deuxième rangée et de la troisième rangée de la microplaque 18a. Le nombre de lavage peut être cependant être quelconque. De même, il a été décrit une seule étape de capture des particules dans les cônes. Une ou plusieurs étapes de libération des particules, chacune suivie d'une nouvelle étape de capture, peuvent être également prévues.

Pour libérer les particules, le champ magnétique de capture des particules est désactivé en retirant la pièce aimantée 16 de son logement, puis des cycles d'aspiration/refoulement d'un tampon est réalisé dans les cônes de pipette de manière à détacher les culots de particules des parois des cônes et les désagréger. Une telle procédure prend plus de 10 minutes pour détacher complètement les culots des parois des cônes avec une fréquence d'aspiration/refoulement (aspiration et refoulement complet dans les cônes) de 5 cycles par minutes. En se référant à la figure 15, une libération plus rapide d'un culot 200 est obtenue en réalisant un mouvement de va-et-vient, sur le culot 200, du ménisque 300 que forme le tampon 302 dans un cône 20. Notamment, le cycle d'aspiration/refoulement est réglé de sorte que le ménisque 300 réalise une course limitée 304 de part et d'autre du culot 200 afin d'augmenter la fréquence de passage du ménisque sur le culot. De même, la fréquence des cycles d'aspiration /refoulement est accrue pour augmenter encore ladite fréquence de passage, en particulier une fréquence cycle supérieure à 2 cycles par secondes sur la zone de présence du culot de particules magnétiques. En appliquant cette procédure, les culots se détachent des cônes en moins de 1 minute. Les inventeurs ont constaté que c'est le passage du ménisque sur un culot qui aide à détacher ce dernier. En effet, des essais ont été menés en brassant rapidement le tampon dans les cônes dans faire passer les ménisques sur les culots (i.e. « simple » mouvement de liquide devant les culots) sans gain notable de temps. Une fois la phase de libération des culots réalisée, qui a également pour effet de désagréger les culots, une phase de brassage par aspiration/refoulement complet dans les cônes est mise en œuvre (e.g. 8 cycles d'aspiration/refoulement par minute) pour terminer de désagréger les culots et homogénéiser le tampon comprenant les particules. Ces cycles d'aspiration/refoulement complets dans les puits de la microplaques brassent un plus grand volume, sur une plus grande course, ce qui facilite l'homogénéisation du tampon.

Il va à présent être décrit un procédé privilégié d'extraction d'acides nucléiques (e.g. ADN et ARN), en particulier d'origine virale, par exemple à l'aide de particules de silice magnétiques. Ce procédé comprend une phase de libération des particules, e.g. telle que décrite précédemment, suivie d'une phase de lavage dans un tampon et de recapture des particules. Un gain notable de temps est obtenu ainsi qu'une extraction améliorée. En particulier, ce procédé comprend, une fois l'étape de lyse des virus effectuée :
1. une première étape de capture des particules magnétiques dans les cônes de pipette, e.g. de la manière décrite précédemment ;
2. suivi d'une première étape de lavage, et de préférence d'au moins une seconde étape de lavage, dans des rangées différentes de la microplaque remplies de tampon de lavage (e.g. le « NucliSENS easyMAG Extraction Buffer n°I » de bioMérieux de référence 280130). Chaque lavage comprend des cycles d'aspiration /refoulement du tampon de lavage avec les particules capturées sur les cônes de pipette, et dure au moins 15 secondes, de préférence entre 25 secondes et 35 secondes, par exemple 30 secondes, et de préférence moins d'une minute ;
3. au moins une troisième étape de lavage dans une troisième rangée de la microplaque 18a remplie d'un tampon de lavage (e.gle « NucliSENS easyMAG Extraction Buffer n°2» de bioMérieux de référence bMx 280131). Lors de cette troisième étape de lavage, les particules sont libérées en retirant l'aimant, de manière à remettre les particules en suspension, le tampon avec les particules en suspension étant aspiré/refoulé dans les puits correspondant de la microplaque 18a. La troisième étape de lavage, de préférence comprenant une phase de passage de ménisques sur les culots telle que décrite précédemment dure quelques minutes, notamment 5 minutes ;
4. une deuxième étape de capture des particules sur les cônes de pipette, e.g. telle que décrite précédemment ;
5. optionnellement une quatrième étape de lavage, les particules étant capturées, dans une troisième rangée de la microplaque 18a remplie d'un tampon de lavage (e.gle « NucliSENS easyMAG Extraction Buffer n°2 » de bioMérieux de référence bMx 280131) ;
6. une étape de migration des culots de particules dans les pointes, suivie d'une étape de transfert dans des tubes (e.g comprenant un tampon d'élution, par exemple le « NucliSENS easyMAG Extraction Buffer n°3 » de bioMérieux, de référence 280132), e.g. tel que décrit précédemment.

Il a été décrit des tampons de lavage de la gamme NucliSens, en particulier des tampons d'extraction n°I, n°2 et n°3. Plus généralement :
- le tampon d'extraction n°I est un tampon favorisant la capture des acides nucléiques sur la silice en créant des ponts entre les groupes silanols de la silice et les groupes phosphates des acides nucléiques. Il comprend par exemple du guanidinium thiocyanate, à savoir un agent chaotropique tel que décrit dans le document de R. Boom et al. "Rapid and simple method for purification ofi nucleic acids." Journal of Clinical Microbiology. 1989; 28 (3): 495-503 ;
- les premier et second lavages permettent d'éliminer les débris résiduels de matrice ou de micro-organismes,
- le troisième et quatrième lavages permettent d'éliminer les traces de GuSCN et des inhibiteurs d'une amplification de type PCR usuellement mise en place ultérieurement sur l'ADN/ARN capturé par les particules magnétiques
- le tampon d'élution compris dans les cônes PCR permet d'enlever toute trace de tampon de lavage et d'être dans les conditions optimales pour l'étape d'élution.

Le tableau suivant compare les résultats obtenus avec le dispositif selon l'invention en appliquant le protocole venant d'être décrit (2 premiers lavage suivi d'un troisième lavage avec libération des particules) en comparaison des résultats obtenus avec un dispositif de l'état de la technique, à savoir le MiniMag® commercialisé par bioMérieux et considéré comme un dispositif de référence dans l'extraction d'ARN viral. Le protocole pour le MiniMag® comprend quatre étapes de lavage avec les tampons de lavage (deux avec le tampon « NucliSENS easyMAG Extraction Buffer n°I » et deux avec le tampon « NucliSENS easyMAG Extraction Buffer n°2 »). Pour déterminer l'efficacité de l'extraction, une amplification PCR en temps réel (ou « q-PCR ») du lysat extrait est mise en œuvre et le Ct (« cycle threshold », qui quantifie un seuil de détection d'acide nucléique dans un échantillon) de chaque échantillon mesuré. Les échantillons testés en duplica sont des échantillon de 25 grammes de framboise ou d'oignon vert auquel sont ajoutés une solution de Mengo virus pure (correspondant à 500 copie du génome par 25 gramme) ou diluée à 1/10^{e}.

| | | Invention (valeur de Ct) | | | | MiniMag® (valeur de Ct) | |
|---|---|---|---|---|---|---|---|
| Echantillon | | framboise | | Oignon vert | | Framboise | Oignon vert |
| Mengo | pur | 25,72 | 26,29 | 25,87 | 25,55 | 24,86 | 25,06 |
| | 1/10 | 27,87 | 28,61 | 28,06 | 27,95 | 28,01 | 27,81 |
| Mengo | pur | 26,05 | 26,11 | 25,82 | 25,87 | 24,93 | 24,99 |
| | 1/10 | 28,23 | 28,44 | 27,59 | 28,13 | 28,06 | 27,76 |

Comme on peut le constater, l'extraction de l'ARN viral selon l'invention donne des résultats similaires avec ceux obtenus à l'aide du MiniMag®. En outre, des tests ont été menés avec des différents lots de particules de silice magnétiques de qualité diverse. Il a été constaté que l'extraction selon l'invention est étonnamment très robuste vis-à-vis de la qualité desdites particules. En particulier, des tests ont été réalisés sur les même échantillons avec un lot de particules de moindre performance, l'extraction ne comportant pas l'étape de libération/lavage/recapture telle que décrite précédemment. Dans ce cas, le taux d'extraction était plus faible. En utilisant le procédé préféré décrit précédemment avec les particules défectueuses, des résultats similaires à ceux de la table précédente ont été obtenus.

Il a été décrit une application de l'invention à la capture d'acides nucléiques, e.g. ARN et/ou ADN provenant d'une lyse réalisée préalablement aux phases de capture/lavage/migration et transfert. L'invention s'applique également à la capture de microorganismes (e.g. bactéries, champignons, levures) à l'aide de particules magnétiques dont la surface est fonctionnalisée pour capturer les microorganismes (e.g. recouvertes de protéines de phage ou de polycations aptes à une telle capture d'une manière connue en soi). Les particules magnétiques avec leur microorganismes capturés sont transférées dans des tubes pour subir ensuite une lyse, par exemple mécanique. Le lysat obtenu peut directement faire l'objet d'un traitement, par exemple une amplification par réaction en chaîne par polymérase (e.g. une PCR quantitative de type q-PCR), ou être purifié selon le procédé d'extraction d'acides nucléiques décrit précédemment.

L'invention est particulièrement adaptée à la préparation d'échantillon microbien en vue d'une PCR. En effet, l'échantillon sur lequel est réalisé la capture de particules dans les cônes de pipette peut être de très grand volume (e.g. plusieurs millilitres) alors que le volume final des tubes dans lesquelles les particules sont transférées peuvent de très faible volume (e.g. inférieur ou égal à 200 micro litres, voire inférieur ou égal à 100 micro litres). En raison du grand volume de l'échantillon, un nombre important de microorganisme est capturé. Le passage à un très faible volume final a pour effet de concentrer les microorganisme. Ainsi, les inventeurs ont noté qu'une seule phase de capture depuis un échantillon de quelques millilitres suivie d'une seule étape de lavage, est suffisant pour obtenir des résultats par q-PCR à partir d'une lyse réalisée dans un volume de 5 microlitres.

Notamment, un enrichissement de matrice alimentaire (aiguillette de poulet) en bouillon nutritif a été réalisé pendant 5h à 41,5°C. Un post-contamination avec une souche Salmonella Derby est réalisé à un niveau de 10<2>à 10<4>CFU/mL, ce qui correspond à des concentrations pouvant être atteintes après enrichissement en présence de pathogène dans la matrice alimentaire (i.e. des concentrations pour lesquelles un lot alimentaire est déterminé comme impropre à la consommation). Sur chaque échantillon contaminé, il est mise en œuvre, en duplica, deux procédures, une selon le protocole standardisé de capture avec le système Gene- up® de bioMérieux, France, et une selon l'invention.

Le protocole du Gene-up® consiste en une étape de « bead-beating » de l'échantillon (i.e. disruption mécanique de la paroi des bactéries), en prélevant 20 de celui-ci que l'on met dans un tube de bead-beating contenant 180 de tampon de lavage, suivi de l'agitation pendant 5 minutes sur agitateur de microplaques pour bead-beating. 5 microlitres de la solution final sont prélevés puis font l'objet d'une q-PCR.

Le procédé selon l'invention consiste quant à lui en :
1. une étape de capture spécifique en mettant en contact 2 mL d'échantillon avec une solution de protéine de phage biotinylée (2 µg/mL finale) par :
a. agitation par aspiration/refoulement dans les cônes de la pipette pendant 10 min,
b. ajout de particules magnétiques « Hyglos Streptavidin » (50 µí) et agitation par aspiration/refoulement pendant 15 minutes (les complexes bactéries-protéines de phage biotynilées viennent se fixer sur les particules magnétiques) ;
c. mise en place de l'aimant pour la phase de collecte des particules magnétiques dans les cônes ;
d. lancement du cycle de capture des particules magnétiques ;

2. une étape de lavage en dans les puits contenant la solution de lavage TST (Tris Saline Tween) avec 5 cycles d'aspiration/refoulement ;
3. étape de collecte des particules magnétiques dans des tubes de 5 micro litres qui font l'objet d'un traitement de bead-Beating, la solution finale de 5 microlitres faisant ensuite l'objet d'une q-PCR. Les résultats obtenus selon le protocole du Gene-up® et selon l'invention sont résumés dans la table ci-dessous:

| Concentration | Invention (valeur de Ct) | Gene-up® (valeur de Ct) |
|---|---|---|
| 10² CFU/ml | 35,2 | Pas de Ct |
| | 35 | Pas de Ct |
| 10³ CFU/ml | 33,8 | Pas de Ct |
| | 33,8 | Pas de Ct |
| 1 0⁴ CFU/ml | 29,5 | 34,9 |
| | 29,7 | Pas de Ct |

Le gain de sensibilité estimé est de 2 log par rapport au protocole standard Gene-up®.

La présente invention répond à une problématique de polyvalence pour l'utilisation de différentes techniques de capture magnétique (purification des acides nucléiques, Immuno- concentration magnétiques,...). Le système selon l'invention, évolutif et modulable, permet :
- la réalisation d'étapes de capture/lavage/élution de particules magnétiques en utilisant un système autonome constitué par l'association d'une pipette électronique programmable et d'un support permettant la réalisation des différentes étapes précitées ;
- le traitement d'un nombre d'échantillon de 1 à 8 en fonction de la configuration de la pipette électronique utilisée ;
- la parallélisation du traitement des échantillons dans le cadre défini ci-dessus avec un système semi-automatique ;
- l'association de 2 systèmes si besoin d'augmenter le nombre d'échantillon à traiter ;
- les étapes d'élution peuvent être réalisées dans différents types de tubes : tubes PCR 0.2 mL pour récupération des particules de silice magnétiques lorsqu'il s'agit de capture des acides nucléiques (e.g. chimie NucliSENS©) ou bien tubes de bead-beating dans le cas de la récupération de particules magnétiques ayant servi à la récupération de pathogènes (Immuno-Concentration Magnétique). A cette fin, le système permet la réalisation d'étapes de capture/concentration des pathogènes sur les particules magnétiques et leur lyse in-situ à l'aide de billes de céramique / verre (e.g. procédé de type CapLyse©).

## Revendications

1. Procédé d'extraction de composants contenus dans un échantillon biologique sous forme liquide, lesdits composants étant aptes à se fixer sur des particules magnétiques, le procédé comprenant :
- une phase (106) de mélange de l'échantillon avec les particules magnétiques;
- une phase (110) d'aspiration du mélange depuis un puits dans un cône de pipette (20) tabulaire comprenant une pointe (21) destinée au pipetage de liquide;
- une phase de capture (112) des particules magnétiques sur une paroi interne du cône de pipette (20) :
- en appliquant un premier champ magnétique (16) au cône de pipette (20), ledit champ étant apte à attirer et maintenir les particules magnétiques dans une zone prédéterminée du cône de pipette (20), dite de « capture », au-dessus de la pointe de celui-ci ;
- et en appliquant au moins un cycle d'aspiration et de refoulement du mélange contenu dans le cône de pipette (20) dans un puits (62);
- au moins une phase (114, 116) de lavage des particules capturées sur la paroi interne du cône de pipette (20) en :
- refoulant le mélange contenu dans le cône de pipette (20) ; et
- en appliquant depuis un puits (62) contenant une solution de lavage au moins un cycle d'aspiration et de refoulement de la solution de lavage dans le cône de pipette (20) ; **caractérisé en ce que** le procédé comprend en outre :
- une phase (118) de migration des particules magnétiques sur la paroi interne du cône de pipette (20), depuis la zone de capture jusqu'à la pointe (21) du cône de pipette (20), en réalisant un déplacement relatif du cône de pipette (20) par rapport au premier champ magnétique (16) ;
- et une phase (120) de transfert desdites particules magnétiques ayant migré dans la pointe (21) du cône de pipette (20) dans un puits de récupération (69) contenant une solution,
- au moins une phase de lavage des particules capturées sur la paroi interne du cône de pipette, préalablement à la phase (120) de transfert, en :
• désactivant le champ magnétique ;
• libérant les particules capturées en appliquant depuis un puits contenant une solution de lavage au moins un cycle d'aspiration et de refoulement de la solution de lavage dans le cône de pipette ;
• appliquant une deuxième phase de capture sur une paroi interne du cône de pipette :
• appliquant le premier champ magnétique au cône de pipette
• appliquant au moins un cycle d'aspiration et de refoulement du mélange contenu dans le cône de pipette dans le puits contenant la solution de lavage.

2. Procédé selon la revendication 1, dans lequel la libération des particules capturées comprend une phase d'application des cycles de manière à réaliser un va-et-vient d'un ménisque de ladite solution sur un culot de particules capturées dans le cône de pipette, ledit va-et-vient dudit ménisque étant réalisé sur une portion du cône inférieure à la longueur totale du cône de pipette.

3. Procédé selon la revendication 2, dans lequel la libération des particules capturées comprend une deuxième phase d'application des cycles de manière à aspirer et refouler totalement la solution de lavage du cône.

4. Procédé selon la revendication 3, dans lequel la fréquence d'application des cycles de la deuxième phase est inférieure à la fréquence d'application des cycles de la première phase.

5. Procédé selon l'une des revendications 1 à 4, dans lequel préalablement à la libération des particules capturées au moins deux phases de lavage mises en œuvre dans deux solutions de lavage distinctes.

6. Procédé selon l'une quelconque des revendications 1 à 5, dans lequel les composants contenus dans l'échantillon biologique sont des acides nucléiques.

## Patentansprüche

1. Verfahren zur Extraktion von in einer biologischen Probe in flüssiger Form enthaltenen Komponenten, wobei sich diese Komponenten an magnetische Partikel binden können, wobei das Verfahren umfasst:
- eine Phase (106) des Mischens der Probe mit den magnetischen Partikeln;
- eine Phase (110) des Absaugens des Gemischs aus einer Vertiefung in einen röhrenförmigen Pipettenkegel (20), der eine zum Pipettieren von Flüssigkeit bestimmte Spitze (21) umfasst;
- eine Phase des Einfangens (112) der magnetischen Partikel auf einer Innenwand des Pipettenkegels (20):
- durch Anlegen eines ersten Magnetfelds (16) an den Pipettenkegel (20), wobei dieses Feld in der Lage ist, die magnetischen Partikel in einer festgelegten, als "Einfangzone" bezeichneten Zone des Pipettenkegels (20) oberhalb von dessen Spitze anzuziehen und festzuhalten;
- sowie durch Anwenden mindestens eines Zyklus von Absaugen und Zurückfließenlassen des im Pipettenkegel (20) enthaltenen Gemischs in eine Vertiefung (62);
- mindestens eine Phase (114, 116) des Waschens der auf der Innenwand des Pipettenkegels (20) eingefangenen Partikel durch:
- Zurückfließenlassen des im Pipettenkegel (20) enthaltenen Gemischs und
- durch Anwenden, aus einer Vertiefung (62), die eine Waschlösung enthält, mindestens eines Zyklus von Absaugen und Zurückfließenlassen der Waschlösung in den Pipettenkegel (20); **dadurch gekennzeichnet, dass** das Verfahren zudem umfasst:
- eine Phase (118) der Wanderung der magnetischen Partikel auf der Innenwand des Pipettenkegels (20) aus der Einfangzone bis zur Spitze (21) des Pipettenkegels (20) mittels Durchführung einer relativen Verlagerung des Pipettenkegels (20) in Bezug auf das erste Magnetfeld (16) ;
- und eine Phase (120) der Überführung der magnetischen Partikel, die in die Spitze (21) des Pipettenkegels (20) gewandert sind, in eine Rückgewinnungsvertiefung (69), die eine Lösung enthält,
- mindestens eine Phase des Waschens der auf der Innenwand des Pipettenkegels eingefangenen Partikel vor der Überführungsphase (120) durch:
• Deaktivieren des Magnetfelds;
• Freisetzen der eingefangenen Partikel durch Anwenden, aus einer Vertiefung, die eine Waschlösung enthält, mindestens eines Zyklus von Absaugen und Zurückfließenlassen der Waschlösung in den Pipettenkegel;
• Anwenden einer zweiten Phase des Einfangens auf der Innenwand des Pipettenkegels:
• Anlegen des ersten Magnetfelds an den Pipettenkegel
• Anwenden mindestens eines Zyklus von Absaugen und Zurückfließenlassen des im Pipettenkegel enthaltenen Gemischs in die Vertiefung, die die Waschlösung enthält.

2. Verfahren nach Anspruch 1, wobei die Freisetzung der eingefangenen Partikel eine Phase des Anwendens von Zyklen umfasst, derart, dass eine Auf- und Abbewegung eines Meniskus der Lösung über ein Pellet von im Pipettenkegel eingefangenen Partikel durchgeführt wird, wobei die Auf- und Abbewegung des Meniskus auf einem Teil des Kegels durchgeführt wird, der kürzer ist als die Gesamtlänge des Pipettenkegels.

3. Verfahren nach Anspruch 2, wobei die Freisetzung der eingefangenen Partikel eine zweite Phase des Anwendens der Zyklen umfasst, derart, dass man die Waschlösung aus dem Kegel vollständig absaugt und wieder zurückfließen lässt.

4. Verfahren nach Anspruch 3, wobei die Häufigkeit der Anwendung der Zyklen der zweiten Phase kleiner ist als die Häufigkeit der Anwendung der Zyklen der ersten Phase.

5. Verfahren nach einem der Ansprüche 1 bis 4, wobei vor der Freisetzung der eingefangenen Partikel mindestens zwei Waschphasen in zwei verschiedenen Waschlösungen durchgeführt werden.

6. Verfahren nach einem der Ansprüche 1 bis 5, wobei es sich bei den in der biologischen Probe enthaltenen Komponenten um Nukleinsäuren handelt.

## Claims

1. A method for extracting components contained in a biological sample in liquid form, said components being capable of binding to magnetic particles, the method comprising:
- a phase (106) of mixing the sample with the magnetic particles;
- a phase (110) of suctioning the mixture from a well in a tubular pipette cone (20) comprising a tip (21) intended for pipetting liquid;
- a phase of capturing (112) the magnetic particles on an internal wall of the pipette cone (20) :
- by applying a first magnetic field (16) to the pipette cone (20), said field being capable of attracting and holding the magnetic particles in a predetermined zone of the pipette cone (2), termed "capture" zone, above the tip of said cone;
- and applying at least one cycle of suction and discharge of the mixture contained in the pipette cone (20) in a well (62);
- at least one phase (114, 116) of washing the particles captured on the internal wall of the pipette cone (20) by:
- discharging the mixture contained in the pipette cone (20); and
- applying, from a well (62) containing a washing solution, at least one cycle of suction and discharge of the washing solution in the pipette cone (20); **characterized in that** the method also comprises:
- a phase (118) of migration of the magnetic particles on the internal wall of the pipette cone (20), from the capture zone to the tip (21) of the pipette cone (20), by carrying out a relative movement of the pipette cone (20) relative to the first magnetic field (16);
- and a phase (120) of transferring said magnetic particles having migrated into the tip (21) of the pipette cone (20) into a recovery well (69) containing a solution,
- at least one phase of washing the particles captured on the internal wall of the pipette cone, prior to the transfer phase (120), by:
• deactivating the magnetic field;
• releasing the captured particles by applying, from a well containing a washing solution, at least one cycle of suction and discharge of the washing solution in the pipette cone;
• applying a second phase of capturing on an internal wall of the pipette cone:
• applying the first magnetic field to the pipette cone,
• applying at least one cycle of suction and discharge of the mixture contained in the pipette cone in the well containing the washing solution.

2. Method according to Claim 1, in which the release of the captured particles comprises a phase of applying the cycles in such a way as to carry out an up and down movement of a meniscus of said solution over a pellet of particles captured in the pipette cone, said up and down movement of said meniscus being carried out on a portion of the cone less than the total length of the pipette cone.

3. Method according to Claim 2, in which the release of the captured particles comprises a second phase of applying the cycles so as to totally discharge the cone washing solution.

4. Method according to Claim 3, in which the frequency of application of the cycles of the second phase is lower than the frequency of application of the cycles of the first phase.

5. Method according to one of Claims 1 to 4, in which, prior to the release of the captured particles, at least two washing phases are carried out in two distinct washing solutions.

6. Method according to any one of Claims 1 to 5, in which the components contained in the biological sample are nucleic acids.
